# EUROPEAN PATENT SPECIFICATION

(11) **EP 4 474 812 B1**
(45) Date of publication and mention of the grant of the patent: **30.09.2026**
(21) Application number: 23213197.9
(22) Date of filing: 30.11.2023
(51) Int. Cl.: G01N 33/00

(54) **DEVICE FOR MEASURING ODOR AND METHOD FOR MEASURING ODOR USING THE SAME**
VORRICHTUNG ZUR MESSUNG VON GERÜCHEN UND VERFAHREN ZUR MESSUNG VON GERÜCHEN DAMIT
DISPOSITIF DE MESURE D'ODEUR ET PROCÉDÉ DE MESURE D'ODEUR L'UTILISANT

(30) Priority: 09.06.2023 KR 20230074366
(43) Date of publication of application: 11.12.2024
(73) Proprietor: HYUNDAI MOTOR COMPANY, Seoul 06797 (KR); Kia Corporation, Seocho-gu Seoul 06797 (KR)
(72) Inventor: LEE, Tae Hee, 18280 Hwaseong-si, Gyeonggi-do, (KR); SUNG, Dae Un, 18280 Hwaseong-si, Gyeonggi-do, (KR)
(74) Representative: Viering, Jentschura & Partner mbB Patent- und Rechtsanwälte

(56) References cited:
- WO-A2-99/47905
- US-A1- 2010 001 211
- US-A1- 2017 184 556
- FAHIM FAHIM ET AL: "Novel SAW CWA Detector Using Temperature Programmed Desorption", IEEE SENSORS JOURNAL, IEEE, USA, vol. 21, no. 5, 8 December 2020 (2020-12-08), pages 5914 - 5922, XP011835766, ISSN: 1530-437X, [retrieved on 20210204], DOI: 10.1109/JSEN.2020.3042766

## Description

### TECHNICAL FIELD

The present disclosure relates to a device and method for measuring an odor.

### BACKGROUND

As is well known, inherent odors and volatile organic compound (VOC) odors produced from various types of interior materials in vehicles such as seat covers, headlining, door trims, mats, and the like are a major cause of complaints in new vehicles.

In addition, mold proliferation and offensive odors, which are caused by moisture condensed on an evaporator core when an air conditioner of the vehicle operates, also cause unpleasantness to vehicle users.

In addition, depending on surrounding environments while the vehicle travels, various types of offensive odors may be contained in outside air introduced from the outside, and the offensive odors introduced from the outside may also cause unpleasantness to the vehicle user.

As described above, various types of odors, which are produced inside and outside the vehicle, may degrade the functional quality of the vehicle.

Therefore, accurate measurement by the odor sensor and the construction of odor data are required in advance to analyze the causes of various types of odors produced inside and outside the vehicle and eliminate the various types of odors. Further, it is necessary to perform a process of analyzing components and concentrations of various types of odors produced inside and outside the vehicle and clearly determining the actual cause of the offensive odors.

Meanwhile, in order to solve offensive odor-complaints that occur in various types of industrial sites as well as vehicles, it is necessary to dispose, in advance, odor sensors in the corresponding industrial sites and accurately measure components and concentrations of odors.

To this end, as the odor sensors in the related art for measuring odors produced in various types of industrial sites as well as vehicles, an electrochemical odor sensor and an electrochemical odor sensor array may be used. Alternatively, a bio-odor sensor, such as a bio-peptide type sensor and a sensor using amino acids, may be used.

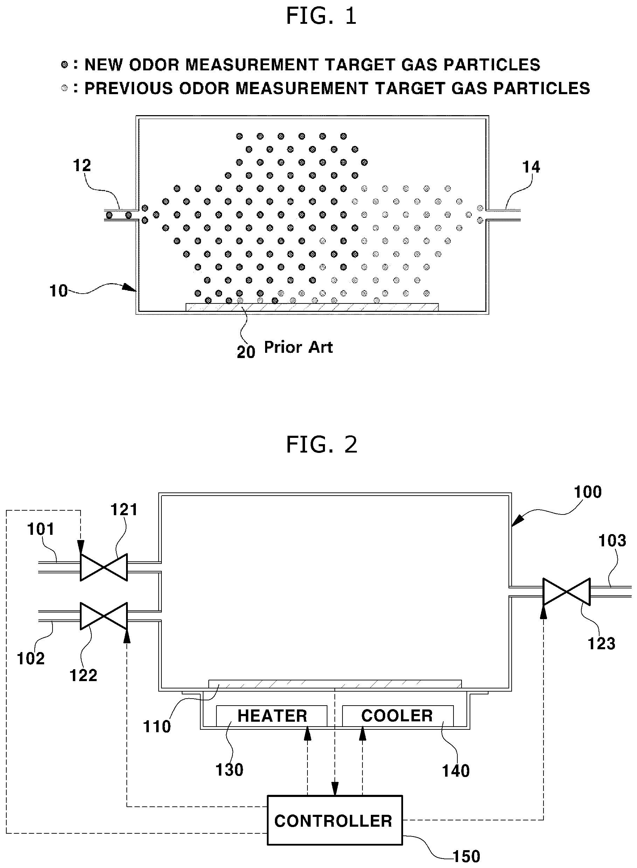

With reference to FIG. 1, when an odor measurement target gas flows from a gas inlet port 12 provided at one side portion of an odor sensor chamber 10 toward a gas discharge port 14 provided at the other side portion in a state in which an odor sensor 20 in the related art is attached in the odor sensor chamber 10, odor measurement target gas particles are adsorbed to a surface of the odor sensor 20, such that the odors may be measured by the odor sensor 20.

For example, the odor measurement may be performed while outputting an electrical signal when the odor measurement target gas particles are adsorbed to the surface of the electrochemical odor sensor. Alternatively, the odor measurement may be performed while outputting different color signals, different RGB signals, and different lightness signals depending on the amount of gas adsorption when the odor measurement target gas particles are adsorbed to the surface of the bio-odor sensor.

However, the odor data measured by the odor sensors are obtained on the basis of a predetermined time. There may be a problem in that the odor measurement target gas particles may not be uniformly mixed in the air for the predetermined time, the odor data measured by the odor sensor may vary depending on the temperature and humidity of the air containing the gas particles, and the measurement accuracy of the odor sensor may eventually deteriorate.

In particular, after the odor measurement target gas particles are adsorbed to the surface of the odor sensor, the odor measurement target gas particles need to be desorbed in order to accurately perform the odor measurement on newly introduced gas particles. However, the previously adsorbed gas particles cannot be properly desorbed from the surface of the odor sensor, which causes a problem in which the measurement accuracy of the odor sensor deteriorates.

In more detail, as illustrated in FIG. 1, when some of the previous odor measurement target gas particles are still kept adsorbed without being desorbed from the surface of the odor sensor 20 mounted in the odor sensor chamber 10, there may occur a problem in that new odor measurement target gas particles are mixed with some of the previous odor measurement target gas particles or cannot be properly adsorbed to the surface of the odor sensor, which inevitably degrades the measurement accuracy of the odor sensor. From WO 99/47905 A2, US 2017/184556 A1, Fahim Fahim et al: "Novel SAW CWA Detector Using Temperature Programmed Desorption", IEEE Sensors Journal, vol. 21, 8 December 2020, pages 5914-5922 and US 2010/001211 A1 devices for measuring odor are known.

The above information disclosed in this background section is only for enhancement of understanding of the background of embodiments of the invention, and therefore it may contain information that does not form the prior art that is already known to a person of ordinary skill in the art.

### SUMMARY

The present disclosure relates to a device and method for measuring an odor. Particular embodiments relate to a device and method for measuring an odor, which are capable of improving measurement accuracy of an odor sensor by allowing gas particles to be easily adsorbed to and desorbed from a surface of the odor sensor.

The present invention provides a device for measuring an odor according to claim 1 to solve problems associated with prior art. The present invention provides a method of measuring an odor according to claim 6 to solve problems associated with prior art. Further embodiments are subject of the dependent claims.

Embodiments of the present disclosure can solve problems in the related art, and embodiments of the present disclosure provide a device and method for measuring an odor that supply cleaning air, such as fresh air or pure air, into an odor sensor chamber after odor measurement performed by an odor sensor attached in an odor sensor chamber is ended, thereby allowing the cleaning air to eliminate previous odor measurement target gas particles remaining in the odor sensor chamber and remaining on a surface of the odor sensor, and heat the odor sensor to easily desorb previous odor measurement target gas particles from the surface of the odor sensor, thereby improving measurement accuracy of the odor sensor.

Another embodiment of the present disclosure provides a device and method for measuring an odor that supply new odor measurement target gas into an odor sensor chamber and cool an odor sensor after the cleaning air eliminates previous odor measurement target gas particles remaining in the odor sensor chamber and remaining on a surface of the odor sensor, thereby easily adsorbing new odor measurement target gas particles to the surface of the odor sensor and improving measurement accuracy of the odor sensor.

An embodiment of the present disclosure provides a device for measuring an odor, the device including an odor sensor chamber having a cleaning air supply port and a gas inlet port provided at one side portion and a gas discharge port provided at the other side portion, an odor sensor mounted in the odor sensor chamber, a first valve mounted in the cleaning air supply port, a second valve mounted in the gas inlet port, a third valve mounted in the gas discharge port, a first heater mounted in the odor sensor chamber to heat the odor sensor, a first cooler mounted in the odor sensor chamber to cool the odor sensor, and a controller configured to perform control to open or close the first valve, the second valve, and the third valve and turn on or off the first heater and the first cooler.

In a preferred embodiment, the controller may be configured to perform control to open the first valve and the third valve and close the second valve to supply cleaning air into the odor sensor chamber after odor measurement by the odor sensor is ended.

In another preferred embodiment, the controller is configured to perform control to turn on the first heater to heat the odor sensor so that previous odor measurement target gas particles may be desorbed from a surface of the odor sensor after the odor measurement by the odor sensor is ended.

In still another preferred embodiment, the controller may be configured to perform control to open the second valve and close the first valve and the third valve to supply new odor measurement target gas into the odor sensor chamber.

In yet another preferred embodiment, the controller may be configured to perform control to turn on the first cooler to cool the odor sensor so that the new odor measurement target gas particles are adsorbed to a surface of the odor sensor.

Another embodiment of the present disclosure provides a method of measuring an odor, the method including ending odor measurement by an odor sensor mounted in an odor sensor chamber, supplying cleaning air into the odor sensor chamber to eliminate previous odor measurement target gas particles, heating an odor sensor by turning on a first heater mounted in the odor sensor chamber to desorb previous odor measurement target gas particles from a surface of the odor sensor, and discharging the previous odor measurement target gas particles, which are desorbed from the surface of the odor sensor, and the previous odor measurement target gas particles, which remain in the odor sensor chamber, to the outside of the odor sensor chamber by using the cleaning air.

In a preferred embodiment, the method may further include cooling the odor sensor by turning on a first cooler mounted in the odor sensor chamber so that new odor measurement target gas particles are adsorbed to the surface of the odor sensor when new odor measurement target gas is supplied into the odor sensor chamber.

Another embodiment of the present disclosure provides a device for measuring an odor, the device including a pre-chamber having a cleaning air supply port and a first gas inlet port provided at one side portion and a first gas discharge port provided at the other side portion, a gas flow tube connected between the cleaning air supply port, the first gas inlet port, and the first gas discharge port, an odor sensor chamber having a second gas inlet port provided at one side portion and a second gas discharge port provided at the other side portion, a connection tube connected between the first gas discharge port and the second gas inlet port, an odor sensor mounted in the odor sensor chamber, a first valve mounted in the cleaning air supply port, a second valve mounted in the first gas inlet port, a third valve mounted in the second gas discharge port, a temperature adjustment part mounted in the pre-chamber and configured to adjust a temperature of gas passing through the gas flow tube, and a controller configured to perform control to open or close the first valve, the second valve, and the third valve and turn on or off the temperature adjustment part.

In a preferred embodiment, the temperature adjustment part may include a second heater mounted in the pre-chamber to heat the gas passing through the gas flow tube and a second cooler mounted in the pre-chamber to cool the gas passing through the gas flow tube.

In another preferred embodiment, the controller may be configured to perform control to open the first valve and the third valve and close the second valve to supply the cleaning air into the odor sensor chamber through the gas flow tube after odor measurement by the odor sensor is ended.

In still another preferred embodiment, the controller may be configured to perform control to turn on the second heater to heat the cleaning air flowing through the gas flow tube.

In yet another preferred embodiment, the controller may be configured to perform control to open the second valve and close the first valve and the third valve to supply new odor measurement target gas into the odor sensor chamber through the gas flow tube.

In still yet another preferred embodiment, the controller may be configured to perform control to turn on the second cooler to cool the new odor measurement target gas flowing through the gas flow tube.

In a further preferred embodiment, the device may further include a first heater mounted in the odor sensor chamber to heat the odor sensor and configured to be controlled to be turned on or off by the controller and a first cooler mounted in the odor sensor chamber to cool the odor sensor and configured to be controlled to be turned on or off by the controller.

In another further preferred embodiment, the controller may be configured to perform control to turn on the first heater to heat the odor sensor so that previous odor measurement target gas particles are desorbed from a surface of the odor sensor after odor measurement by the odor sensor is ended.

In still another further preferred embodiment, the controller may be configured to perform control to turn on the first cooler to cool the odor sensor so that new odor measurement target gas particles are adsorbed to a surface of the odor sensor.

Another embodiment of the present disclosure provides a method of measuring an odor, the method including ending odor measurement by an odor sensor mounted in an odor sensor chamber, supplying cleaning air to a gas flow tube of a pre-chamber connected to communicate with the odor sensor chamber, heating cleaning air flowing through the gas flow tube by turning on a second heater mounted in the pre-chamber, desorbing previous odor measurement target gas particles from a surface of the odor sensor by introducing the heated cleaning air into the odor sensor chamber, and discharging previous odor measurement target gas particles, which are desorbed from the surface of the odor sensor, and the previous odor measurement target gas particles, which remain in the odor sensor chamber, to the outside of the odor sensor chamber by using the cleaning air.

In a preferred embodiment, the method may further include heating the odor sensor by turning on a first heater mounted in the odor sensor chamber so that the previous odor measurement target gas particles are desorbed from the surface of the odor sensor.

In another preferred embodiment, the method may further include cooling new odor measurement target gas flowing through the gas flow tube by turning on a second cooler mounted in the pre-chamber so that new odor measurement target gas particles are adsorbed to the surface of the odor sensor when the new odor measurement target gas is supplied into the odor sensor chamber through the gas flow tube of the pre-chamber.

In still another preferred embodiment, the method may further include cooling the odor sensor by turning on a first cooler mounted in the odor sensor chamber so that the new odor measurement target gas particles are adsorbed to the surface of the odor sensor.

Embodiments of the present disclosure provide the following effects through the above-mentioned solutions.

First, the previous odor measurement target gas particles remaining in the odor sensor chamber and remaining on the surface of the odor sensor may be eliminated by the cleaning air, and the odor sensor may be heated to easily desorb the previous odor measurement target gas particles from the surface of the odor sensor, thereby improving the measurement accuracy of the odor sensor.

Second, the cleaning air may be heated in advance in the pre-chamber and then supplied into the odor sensor chamber, such that the previous odor measurement target gas particles may be more easily desorbed from the surface of the odor sensor by the heated cleaning air, thereby further improving the measurement accuracy of the odor sensor.

Third, the odor sensor may be cooled after the previous odor measurement target gas particles remaining in the odor sensor chamber and remaining on the surface of the odor sensor are eliminated, such that the new odor measurement target gas particles may be easily adsorbed to the surface of the odor sensor, thereby improving the measurement accuracy of the odor sensor.

Fourth, the cleaning air may be cooled in advance in the pre-chamber and then supplied into the odor sensor chamber, such that the new odor measurement target gas particles may be more easily adsorbed to the surface of the odor sensor by the cooled cleaning air, thereby further improving the measurement accuracy of the odor sensor.

Other features of preferred embodiments of the invention are discussed infra.

It is understood that the term "vehicle" or "vehicular" or other similar term as used herein is inclusive of motor vehicles in general such as passenger automobiles including sport utility vehicles (SUVs), buses, trucks, various commercial vehicles, watercraft including a variety of boats and ships, aircraft, and the like, and includes hybrid vehicles, electric vehicles, plug-in hybrid electric vehicles, hydrogen-powered vehicles, and other alternative fuel vehicles (e.g., fuels derived from resources other than petroleum). As referred to herein, a hybrid vehicle is a vehicle that has two or more sources of power, for example both gasoline-powered and electric-powered vehicles.

The above and other features of embodiments of the invention are discussed infra.

### BRIEF DESCRIPTION OF THE DRAWINGS

The above and other features of embodiments of the present disclosure will now be described in detail with reference to certain exemplary embodiments thereof illustrated in the accompanying drawings which are given hereinbelow by way of illustration only, and thus are not limitative of the embodiments of the present disclosure, and wherein:
FIG. 1 is a schematic view illustrating an odor measurement device in the related art;
FIG. 2 is a schematic view illustrating an odor measurement device according to an embodiment of the present disclosure;
FIGS. 3, 4, and 5 are schematic views sequentially illustrating operation flows of the odor measurement device according to an embodiment of the present disclosure;
FIG. 6 is a flowchart illustrating an odor measurement method according to an embodiment of the present disclosure;
FIG. 7 is a schematic view illustrating an odor measurement device according to another embodiment of the present disclosure;
FIGS. 8, 9, and 10 are schematic views sequentially illustrating operation flows of the odor measurement device according to another embodiment of the present disclosure; and
FIG. 11 is a flowchart illustrating an odor measurement method according to another embodiment of the present disclosure.

It should be understood that the appended drawings are not necessarily to scale, presenting a somewhat simplified representation of various preferred features illustrative of the basic principles of embodiments of the invention. The specific design features of embodiments of the present disclosure as disclosed herein, including, for example, specific dimensions, orientations, locations, and shapes, will be determined in part by the particular intended application and use environment.

In the figures, reference numbers refer to the same or equivalent parts of the present disclosure throughout the several figures of the drawings.

### DETAILED DESCRIPTION OF ILLUSTRATIVE EMBODIMENTS

Hereinafter reference will now be made in detail to various embodiments of the present disclosure, examples of which are illustrated in the accompanying drawings and described below. While embodiments of the invention will be described in conjunction with exemplary embodiments, it will be understood that the present description is not intended to limit the embodiments of the invention to those exemplary embodiments. On the contrary, the embodiments of the invention are intended to cover not only the exemplary embodiments, but also various alternatives, modifications, and other embodiments, which are included within the invention as defined by the appended claims.

Specific structural or functional descriptions described in embodiments of the present specification are exemplified only for the purpose of explaining the embodiments according to the concept of the present disclosure, and the embodiments according to the concept of the present disclosure may be carried out in various forms. In addition, embodiments of the present disclosure should not be interpreted as being limited to the embodiments disclosed in the present specification, and it should be understood that the embodiments of the present disclosure include all modifications, and alternatives included in the invention as defined by the appended claims.

Meanwhile, the terms such as "first" and/or "second" in the present disclosure may be used to describe various constituent elements, but these constituent elements should not be limited by these terms. These terms are used only for the purpose of distinguishing one constituent element from other constituent elements. For example, without departing from the scope according to the concept of the present disclosure, a first constituent element may be referred to as a second constituent element, and similarly, the second constituent element may also be referred to as the first constituent element.

When one constituent element is described as being "coupled" or "connected" to another constituent element, it should be understood that one constituent element can be coupled or connected directly to another constituent element, and an intervening constituent element can also be present between the constituent elements. When one constituent element is described as being "coupled directly to" or "connected directly to" another constituent element, it should be understood that no intervening constituent element is present between the constituent elements. Other expressions, that is, "between" and "just between" or "adjacent to" and "directly adjacent to", for explaining a relationship between constituent elements, should be interpreted in a similar manner.

Like reference numerals indicate like constituent elements throughout the specification. The terms used in the present specification are for explaining the exemplary embodiments, not for limiting the present disclosure. Unless particularly stated otherwise in the present specification, a singular form also includes a plural form. The terms "comprise (include)" and/or "comprising (including)" used in the specification are intended to specify the presence of the mentioned constituent elements, steps, operations, and/or elements, but do not exclude the presence or addition of one or more other constituent elements, steps, operations, and/or elements.

Hereinafter, exemplary embodiments of the present disclosure will be described in detail with reference to the accompanying drawings.

### First Embodiment

FIGS. 2 to 5 illustrate an odor measurement device according to an embodiment of the present disclosure.

An odor sensor chamber 100 has a structure in which a cleaning air supply port 101 and a gas inlet port 102 are provided at one side portion and a gas discharge port 103 is provided at the other side portion.

In particular, an odor sensor 110 is attached to a bottom surface in the odor sensor chamber 100. The odor sensor 110 may be an electrochemical odor sensor or a bio-odor sensor.

Therefore, the odor measurement may be performed by the odor sensor 110 as odor measurement target gas particles are adsorbed to a surface of the odor sensor 110.

For example, the odor measurement may be performed while outputting an electrical signal to a controller 150 when the odor measurement target gas particles are adsorbed to the surface of the electrochemical odor sensor. Alternatively, the odor measurement may be performed while outputting color signals, RGB signals, and lightness signals to the controller 150 when the odor measurement target gas particles are adsorbed to the surface of the bio-odor sensor.

A cleaning air supply source may be connected to the cleaning air supply port 101 of the odor sensor chamber 100 to supply cleaning air such as fresh air (filtered outside air) or pure air (e.g., pure nitrogen, pure carbon dioxide, or the like).

The gas inlet port 102 of the odor sensor chamber 100 is connected to and communicates with a vehicle interior, such that air containing various types of odors produced inside and outside the vehicle or future mobility vehicles (PBVs or UAMs) may flow into the odor sensor chamber 100 through the gas inlet port 102.

The gas discharge port 103 of the odor sensor chamber 100 is a passageway that communicates with outside air. The gas of which the odor measurement is completed by the odor sensor 110 may be discharged to the outside through the gas discharge port 103.

In addition, the cleaning air supply port 101 is mounted with a first valve 121 that is controlled to be opened at the time of supplying cleaning air into the odor sensor chamber 100. The gas inlet port 102 is mounted with a second valve 122 that is controlled to be opened at the time of supplying odor measurement target gas into the odor sensor chamber 100.

In addition, the gas discharge port 103 is mounted with a third valve 123. The third valve 123 is controlled to be closed so that the odor measurement target gas remains in the odor sensor chamber 100 during the odor measurement by the odor sensor 110. The third valve 123 is controlled to be opened so that the gas is discharged to the outside after the odor measurement is completed by the odor sensor 110.

In particular, a first heater 130 for heating the odor sensor 110 and a first cooler 140 for cooling the odor sensor 110 are mounted side by side at a lower side of the odor sensor chamber 100.

For example, a heating wire coil or the like may be used as the first heater 130. A blower or the like for sucking cold air produced by an operation of an air conditioner of the vehicle may be used as the first cooler 140. Alternatively, a single Peltier element for a heating or cooling process may be used for the first heater 130 and the first cooler 140.

Meanwhile, the odor measurement target gas particles collide and come into contact with the surface of the odor sensor less frequently at a high temperature at a predetermined level or higher, which reduces a rate of the adsorption of the odor measurement target gas particles to the surface of the odor sensor. This is because an adsorption binding force of the gas particles to the surface of the odor sensor decreases as the momentum of the gas particles increases due to thermal vibration.

Therefore, at a high temperature at a predetermined level or higher, the odor measurement target gas particles may not be easily adsorbed to the surface of the odor sensor 110, but the odor measurement target gas particles may be easily desorbed from the surface of the odor sensor 110.

On the contrary, the odor measurement target gas particles collide and come into contact with the surface of the odor sensor more frequently at a low temperature at the predetermined level or lower, and the rate of the adsorption of the odor measurement target gas particles to the surface of the odor sensor increases. This is because an adsorption binding force of the gas particles to the surface of the odor sensor increases as the momentum decreases due to thermal vibration of the gas particles.

Therefore, at a low temperature at the predetermined level or lower, the odor measurement target gas particles may be easily adsorbed to the surface of the odor sensor 110, and the odor measurement target gas particles may not be easily desorbed from the surface of the odor sensor 110.

Therefore, in the case that the previous odor measurement target gas particles are kept adsorbed to the surface of the odor sensor 110 without being desorbed, the first heater 130 is turned on to heat the odor sensor 110, such that the previous odor measurement target gas particles may be easily desorbed from the surface of the odor sensor 110.

In contrast, in the case that new odor measurement target gas is introduced into the odor sensor chamber 100, the first cooler 140 is turned on to cool the odor sensor 110, such that the new odor measurement target gas particles may be easily adsorbed to the surface of the odor sensor 110.

Meanwhile, the controller 150 is configured to control and open or close the first valve 121, the second valve 122, and the third valve 123 and control and turn on or off the first heater 130 and the first cooler 140.

For example, after the odor measurement by the odor sensor 110 is ended, i.e., after the controller 150 receives an odor measurement signal from the odor sensor 110, the controller 150 performs control to open the first valve 121 and the third valve 123 and close the second valve 122 in order to supply the cleaning air into the odor sensor chamber 100.

In addition, after the odor measurement by the odor sensor 110 is ended, i.e., after the controller 150 receives an odor measurement signal from the odor sensor 110, the controller 150 performs control to turn on the first heater 130 to heat the odor sensor 110 so that the previous odor measurement target gas particles are easily desorbed from the surface of the odor sensor 110.

In addition, the controller 150 performs control to turn off the first heater 130 after a predetermined time, and the controller 150 performs control to open the second valve 122 and close the first valve 121 and the third valve 123 to supply new odor measurement target gas into the odor sensor chamber 100.

In addition, the controller 150 is configured to perform control to turn on the first cooler 140 to cool the odor sensor 110 so that the new odor measurement target gas particles, which are introduced into the odor sensor chamber 100, are easily adsorbed to the surface of the odor sensor 110.

In this case, an odor measurement method according to an embodiment of the present disclosure, which is implemented on the basis of the above-mentioned configuration, will be described below.

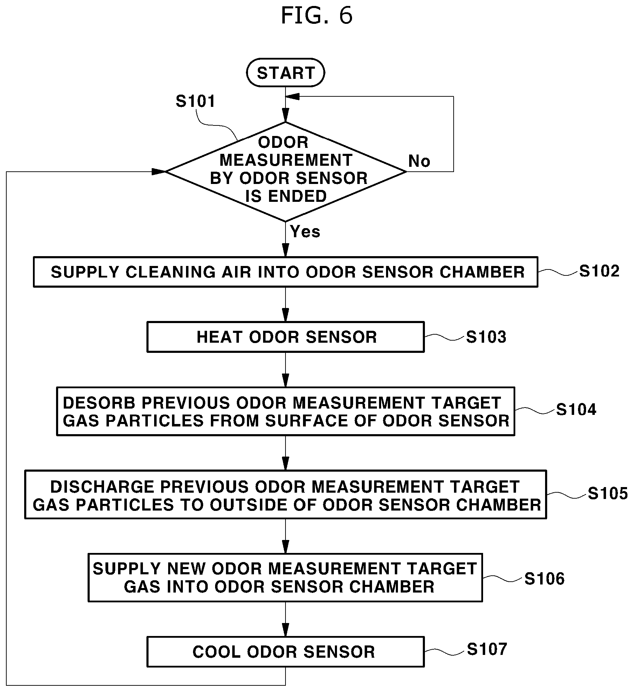

FIG. 6 is a flowchart illustrating an odor measurement method according to an embodiment of the present disclosure.

First, the controller 150 determines whether the odor measurement by the odor sensor 110 mounted in the odor sensor chamber 100 is ended (S101).

For example, the controller 150 may determine that the odor measurement by the odor sensor 110 is ended when the controller 150 receives the odor measurement signal from the odor sensor 110.

In this case, some of the previous odor measurement target gas particles may remain in the odor sensor chamber 100, and some of the previous odor measurement target gas particles may also be kept adsorbed to the surface of the odor sensor 110.

When the determination result in step S101 indicates that the odor measurement by the odor sensor 110 is ended, a step of supplying the cleaning air into the odor sensor chamber 100 to eliminate the previous odor measurement target gas particles is performed (S102).

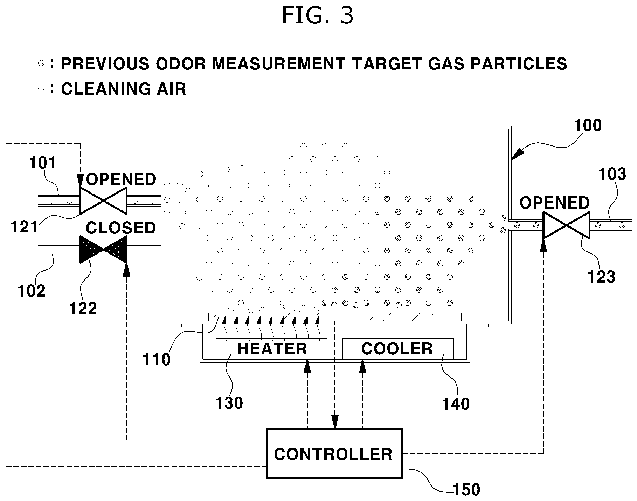

To this end, the controller 150 performs control to open the first valve 121 and the third valve 123 and close the second valve 122, such that the cleaning air provided from the cleaning air supply source may be supplied into the odor sensor chamber 100 while passing through the cleaning air supply port 101 of the odor sensor chamber 100, as illustrated in FIG. 3.

Particularly, the controller 150 performs fuzzy control or on/off control on a pump of the cleaning air supply source on the basis of information on a volume of the odor sensor chamber 100 and information on a flow rate of the cleaning air determined in accordance with a capacity of the pump of the cleaning air supply source configured to supply the cleaning air, such that the cleaning air may be supplied into the odor sensor chamber 100 at a flow rate corresponding to the volume of the odor sensor chamber 100.

In addition, when the determination result in step S101 indicates that the odor measurement by the odor sensor 110 is ended, a step of heating the odor sensor 110 to desorb the previous odor measurement target gas particles from the surface of the odor sensor 110 is performed (S103).

To this end, the controller 150 performs control to turn on the first heater 130, such that the odor sensor 110 may be heated, as illustrated in FIG. 3.

In this case, when the odor sensor 110 is heated, the previous odor measurement target gas particles may be easily desorbed from the surface of the odor sensor 110 (S104). This is because the adsorption binding force of the gas particles to the surface of the odor sensor decreases as the momentum of the gas particles increases due to thermal vibration, as described above.

Moreover, the previous odor measurement target gas particles may be more easily desorbed from the surface of the odor sensor 110 by a flow force of the cleaning air flowing in the odor sensor chamber 100.

Therefore, the previous odor measurement target gas particles, which remain in the odor sensor chamber 100, and the previous odor measurement target gas particles, which are desorbed from the surface of the odor sensor 110, may be discharged to the outside through the gas discharge port 103 of the odor sensor chamber 100 by the flow force of the cleaning air flowing in the odor sensor chamber 100 (S105).

Next, new odor measurement target gas is supplied into the odor sensor chamber 100 (S106).

To this end, the controller 150 performs control to turn off the first heater 130 after a predetermined time, and the controller 150 performs control to open the second valve 122 and close the first valve 121 and the third valve 123 to supply new odor measurement target gas into the odor sensor chamber 100.

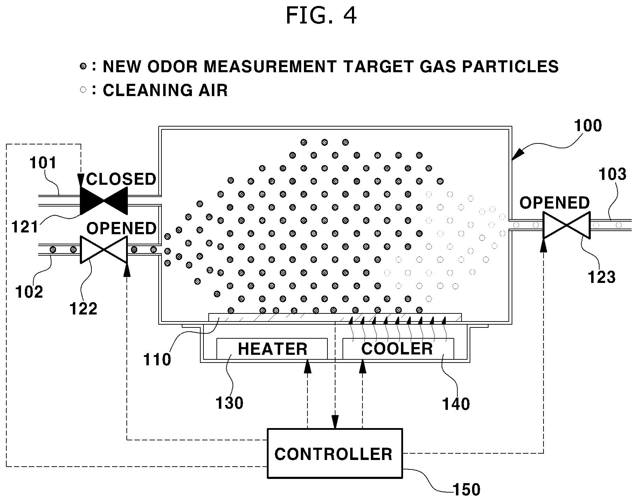

Therefore, as illustrated in FIG. 4, the new odor measurement target gas may be supplied into the odor sensor chamber 100 through the gas inlet port 102 provided at one side portion of the odor sensor chamber 100.

Particularly, the controller 150 performs fuzzy control or on/off control on the pump for supplying the new odor measurement target gas on the basis of information on a volume of the odor sensor chamber 100 and information on a flow rate of the new odor measurement target gas determined in accordance with a capacity of the pump configured to supply the new odor measurement target gas, such that the new odor measurement target gas may be supplied into the odor sensor chamber 100 at a flow rate corresponding to the volume of the odor sensor chamber 100.

In addition, a step of cooling the odor sensor 110 is performed when the new odor measurement target gas is supplied into the odor sensor chamber 100 (S107).

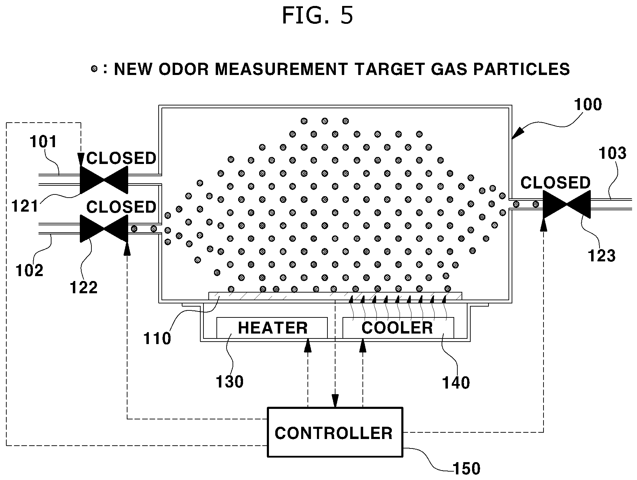

To this end, the controller 150 performs control to turn on the first cooler 140 when performing control to open the second valve 122, such that the odor sensor 110 may be cooled, as illustrated in FIGS. 4 and 5.

In this case, when the odor sensor 110 is cooled, the new odor measurement target gas particles may be easily adsorbed to the surface of the odor sensor 110. This is because the adsorption binding force of the gas particles to the surface of the odor sensor increases as the momentum of the gas particles decreases due to thermal vibration, as described above.

Therefore, the process of cooling the odor sensor 110 allows the new odor measurement target gas particles to be easily adsorbed to the surface of the odor sensor 110, such that the measurement accuracy of the odor sensor may be improved.

Particularly, after the new odor measurement target gas particles are stably adsorbed to the surface of the odor sensor 110 as the controller 150 performs control to turn on the first cooler 140 for a predetermined time, the controller 150 performs control to close the first valve 121, the second valve 122, and the third valve 123, as illustrated in FIG. 5, such that only the new odor measurement target gas remains in the odor sensor chamber 100, and the odor measurement may be accurately performed by the odor sensor 110.

According to an embodiment of the present disclosure described above, the cleaning air is supplied into the odor sensor chamber 100, such that the cleaning air eliminates the previous odor measurement target gas particles remaining in the odor sensor chamber 100 and remaining on the surface of the odor sensor 110. The process of heating the odor sensor 110 allows the previous odor measurement target gas particles to be easily desorbed from the surface of the odor sensor 110. Next, the process of cooling the odor sensor 110 while supplying the new odor measurement target gas into the odor sensor chamber 100 allows the new odor measurement target gas particles to be easily adsorbed to the surface of the odor sensor 110. Therefore, it is possible to improve the measurement accuracy of the odor sensor.

### Second Embodiment

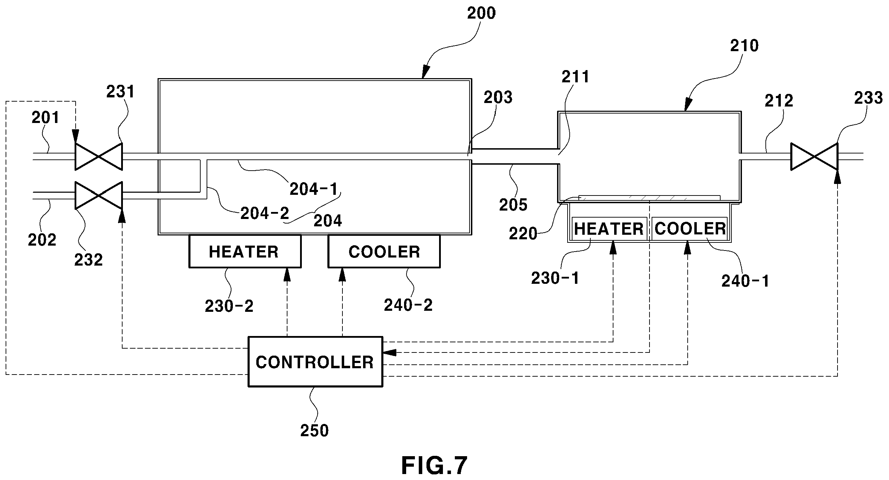

FIGS. 7 to 10 illustrate an odor measurement device according to another embodiment of the present disclosure.

A pre-chamber 200 has a structure in which a cleaning air supply port 201 and a first gas inlet port 202 are provided at one side portion, and a first gas discharge port 203 is provided at the other side portion.

In addition, gas flow tubes 204 are arranged in the pre-chamber 200 and connected between the cleaning air supply port 201, the first gas inlet port 202, and the first gas discharge port 203 to allow cleaning air or odor measurement target gas to flow therethrough.

Particularly, the gas flow tubes 204 may include a first gas flow tube 204-1 connected between the cleaning air supply port 201 and the first gas discharge port 203 and a second gas flow tube 204-2 connected between the first gas inlet port 202 and the first gas flow tube 204-1.

A cleaning air supply source may be connected to the cleaning air supply port 201 of the pre-chamber 200 to supply cleaning air such as fresh air (filtered outside air) or pure air (e.g., pure nitrogen, pure carbon dioxide, or the like).

The first gas inlet port 202 of the pre-chamber 200 is connected to and communicates with a vehicle interior, such that air containing various types of odors produced inside and outside the vehicle or future mobility vehicles (PBVs or UAMs) may flow into the gas flow tubes 204 of the pre-chamber 200 through the first gas inlet port 202.

In addition, an odor sensor chamber 210 is connected to and communicates with the first gas discharge port 203 of the pre-chamber 200 by way of a connection tube 205.

The odor sensor chamber 210 has a structure in which a second gas inlet port 211 is provided at one side portion and a second gas discharge port 212 is provided at the other side portion.

Therefore, the connection tube 205 is connected between the first gas discharge port 203 of the pre-chamber 200 and the second gas inlet port 211 of the odor sensor chamber 210, such that the gas flow tube 204 in the pre-chamber 200 communicates with the odor sensor chamber 210.

In particular, an odor sensor 220 is attached to a bottom surface in the odor sensor chamber 210. The odor sensor 220 may be an electrochemical odor sensor or a bio-odor sensor.

Therefore, the odor measurement may be performed by the odor sensor 220 as odor measurement target gas particles are adsorbed to a surface of the odor sensor 220.

For example, the odor measurement may be performed while outputting an electrical signal to a controller 250 when the odor measurement target gas particles are adsorbed to the surface of the electrochemical odor sensor. Alternatively, the odor measurement may be performed while outputting different color signals, different RGB signals, and different lightness signals to the controller 250 when the odor measurement target gas particles are adsorbed to the surface of the bio-odor sensor.

Meanwhile, the second gas discharge port 212 of the odor sensor chamber 210 is a passageway that communicates with outside air. The gas of which the odor measurement is completed by the odor sensor 220 may be discharged to the outside through the second gas discharge port 212.

The cleaning air supply port 201 of the pre-chamber 200 is mounted with a first valve 231 that is controlled to be opened at the time of supplying cleaning air into the odor sensor chamber 210. The first gas inlet port 202 is mounted with a second valve 232 that is controlled to be opened at the time of supplying odor measurement target gas into the odor sensor chamber 210.

In addition, the second gas discharge port 212 of the odor sensor chamber 210 is mounted with a third valve 233. The third valve 233 is controlled to be closed so that the odor measurement target gas remains in the odor sensor chamber 210 during the odor measurement by the odor sensor 220. The third valve 233 is controlled to be opened so that the gas is discharged to the outside after the odor measurement is completed by the odor sensor 220.

In particular, as temperature adjustment parts mounted in the pre-chamber 200 and configured to adjust a temperature of the gas passing through the gas flow tube 204, a second heater 230-2 is mounted to heat, in advance, the gas passing through the gas flow tube 204 and a second cooler 240-2 is mounted to cool, in advance, the gas passing through the gas flow tube 204.

For example, a heating wire coil or the like may be used as the second heater 230-2. A blower or the like for sucking cold air produced by the operation of the air conditioner of the vehicle may be used as the second cooler 240-2. Alternatively, a single Peltier element for a heating or cooling process may be used for the second heater 230-2 and the second cooler 240-2.

In addition, a first heater 230-1 for heating the odor sensor 220 and a first cooler 240-1 for cooling the odor sensor 220 may be mounted side by side at a lower side of the odor sensor chamber 210.

Likewise, a heating wire coil or the like may be used as the first heater 230-1. A blower or the like for sucking cold air produced by the operation of the air conditioner of the vehicle may be used as the first cooler 240-1. Alternatively, a single Peltier element for a heating or cooling process may be used for the first heater 230-1 and the first cooler 240-1.

Meanwhile, the odor measurement target gas particles collide and come into contact with the surface of the odor sensor less frequently at a high temperature at a predetermined level or higher, which reduces a rate of the adsorption of the odor measurement target gas particles to the surface of the odor sensor. This is because an adsorption binding force of the gas particles to the surface of the odor sensor decreases as the momentum of the gas particles increases due to thermal vibration.

Therefore, at a high temperature at a predetermined level or higher, the odor measurement target gas particles may not be easily adsorbed to the surface of the odor sensor 220, but the odor measurement target gas particles may be easily desorbed from the surface of the odor sensor 220.

On the contrary, the odor measurement target gas particles collide and come into contact with the surface of the odor sensor more frequently at a low temperature at the predetermined level or lower, and the rate of the adsorption of the odor measurement target gas particles to the surface of the odor sensor increase. This is because an adsorption binding force of the gas particles to the surface of the odor sensor increases as the momentum decreases due to thermal vibration of the gas particles.

Therefore, at a low temperature at the predetermined level or lower, the odor measurement target gas particles may be easily adsorbed to the surface of the odor sensor 220, and the odor measurement target gas particles may not be easily desorbed from the surface of the odor sensor 220.

Therefore, in the case that the previous odor measurement target gas particles are kept adsorbed to the surface of the odor sensor 220 without being desorbed, the second heater 230-2 is turned on so that the cleaning air is heated in advance and supplied into the odor sensor chamber 210, and further the first heater 230-1 is turned on to heat the odor sensor 220 so that the previous odor measurement target gas particles may be easily desorbed from the surface of the odor sensor 220.

In contrast, in the case that the new odor measurement target gas is introduced into the odor sensor chamber 210, the second cooler 240-2 is turned on so that the new odor measurement target gas is cooled in advance and supplied into the odor sensor chamber 210, and further the first cooler 240-1 is turned on to cool the odor sensor 220 so that the new odor measurement target gas particles may be easily adsorbed to the surface of the odor sensor 220.

Meanwhile, the controller 250 is configured to control and open or close the first valve 231, the second valve 232, and the third valve 233, control and turn on or off the first heater 230-1 and the first cooler 240-1, and control and turn on or off the second heater 230-2 and the second cooler 240-2.

For example, after the odor measurement by the odor sensor 220 is ended, i.e., after the controller 250 receives an odor measurement signal from the odor sensor 220, the controller 250 performs control to open the first valve 231 and the third valve 233 and close the second valve 232 in order to supply the cleaning air into the odor sensor chamber 210 through the gas flow tube 204.

In addition, after the odor measurement by the odor sensor 220 is ended, i.e., after the controller 250 receives an odor measurement signal from the odor sensor 220, the controller 250 performs control to turn on the second heater 230-2 so that the cleaning air flowing through the gas flow tube 204 is heated in advance.

In addition, after the odor measurement by the odor sensor 220 is ended, i.e., after the controller 250 receives an odor measurement signal from the odor sensor 220, the controller 250 performs control to turn on the first heater 230-1 to heat the odor sensor 220 so that the previous odor measurement target gas particles are desorbed from the surface of the odor sensor 220.

In contrast, the controller 250 performs control to open the second valve 232 and close the first valve 231 and the third valve 233 to supply the new odor measurement target gas into the odor sensor chamber 210 through the gas flow tube 204.

In addition, the controller 250 is configured to perform control to turn on the second cooler 240-2 to cool in advance the new odor measurement target gas flowing through the gas flow tube 204.

In addition, the controller 250 is configured to perform control to turn on the first cooler 240-1 to cool the odor sensor 220 so that the new odor measurement target gas particles are adsorbed to the surface of the odor sensor 220.

In this case, an odor measurement method according to another embodiment of the present disclosure, which is implemented on the basis of the above-mentioned configuration, will be described below.

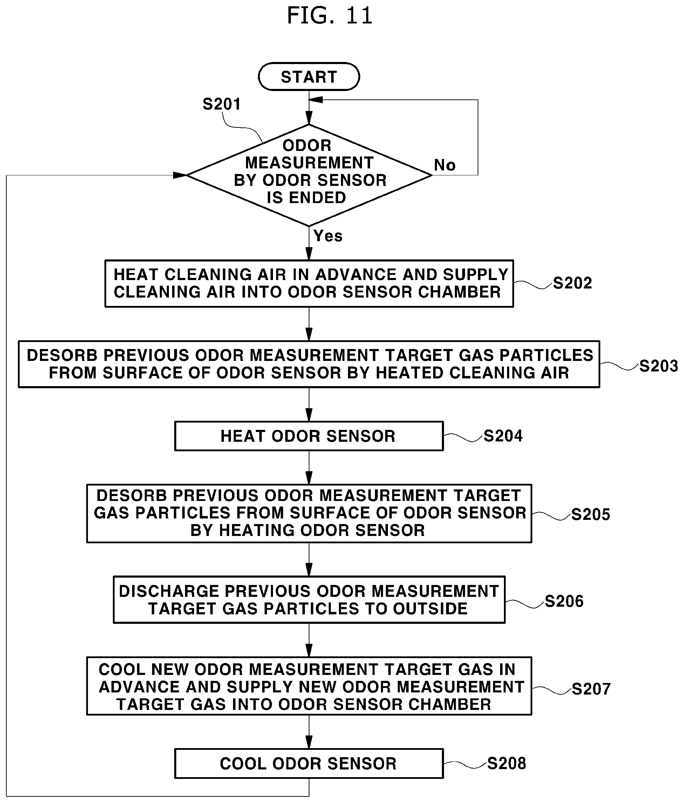

FIG. 11 is a flowchart illustrating an odor measurement method according to another embodiment of the present disclosure.

First, the controller 250 determines whether the odor measurement by the odor sensor 220 mounted in the odor sensor chamber 210 is ended (S201).

For example, the controller 250 may determine that the odor measurement by the odor sensor 220 is ended when the controller 250 receives the odor measurement signal from the odor sensor 220.

In this case, some of the previous odor measurement target gas particles may remain in the odor sensor chamber 210 and some of the previous odor measurement target gas particles may also be kept adsorbed to the surface of the odor sensor 220.

When the determination result in step S201 indicates that the odor measurement by the odor sensor 220 is ended, a step of heating in advance the cleaning air, which is introduced into the cleaning air supply port 201 of the pre-chamber 200 and flows through the gas flow tube 204, and supplying the cleaning air into the odor sensor chamber 210 is performed (S202).

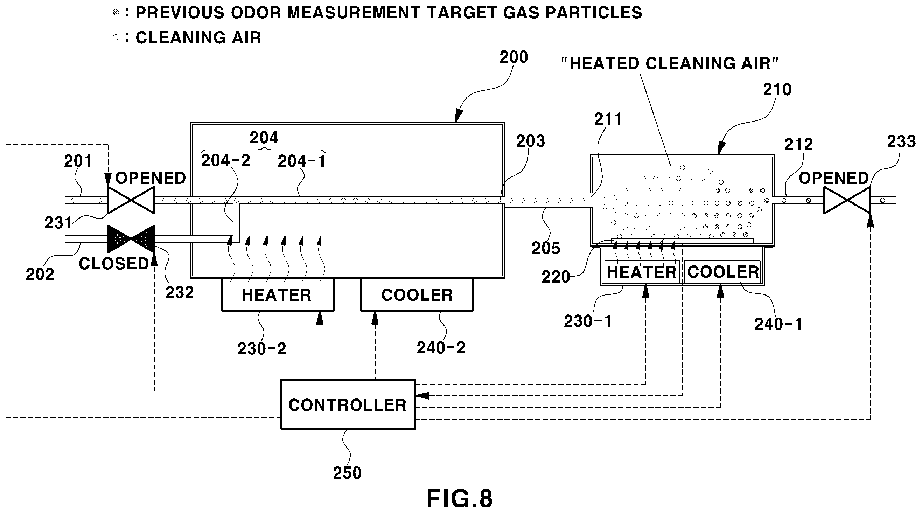

To this end, the controller 250 performs control to open the first valve 231 and the third valve 233 and close the second valve 232, such that the cleaning air provided from the cleaning air supply source may pass through the cleaning air supply port 201 of the pre-chamber 200 and flow along the gas flow tube 204, as illustrated in FIG. 8.

Particularly, the controller 150 performs fuzzy control or on/off control on the pump of the cleaning air supply source on the basis of information on a volume of the gas flow tube 204 of the pre-chamber 200, information on the volume of the odor sensor chamber 100, and information on a flow rate of the cleaning air determined in accordance with a capacity of the pump of the cleaning air supply source configured to supply the cleaning air, such that the cleaning air may be supplied into the odor sensor chamber 100 along the gas flow tube 204 at a flow rate corresponding to the volume of the gas flow tube 204 of the pre-chamber 200 and the volume of the odor sensor chamber 100.

In addition, the controller 250 performs control to turn on the second heater 230-2 mounted in the pre-chamber 200 when performing control to open the first valve 231, such that the cleaning air flowing along the gas flow tube 204 may be heated, and the heated cleaning air may pass sequentially through the first gas discharge port 203, the connection tube 205, and the second gas inlet port 211 and be supplied into the odor sensor chamber 210.

Therefore, when the heated cleaning air is supplied into the odor sensor chamber 210, a function of primarily desorbing the previous odor measurement target gas particles from the surface of the odor sensor 220 is performed (S203).

In more detail, when the heated cleaning air is introduced into the odor sensor chamber 21 and transfers heat to the previous odor measurement target gas particles adsorbed to the surface of the odor sensor 220, the momentum of the gas particles increases due to thermal vibration, and the adsorption binding force of the gas particles to the surface of the odor sensor 220 decreases, such that the previous odor measurement target gas particles may be easily desorbed from the surface of the odor sensor 220. Further, a flow force of the heated cleaning air is applied to the surface of the odor sensor 220, such that the previous odor measurement target gas particles may be easily desorbed from the surface of the odor sensor 220.

Meanwhile, a step of heating the odor sensor 220 may be further performed to more easily desorb the previous odor measurement target gas particles from the surface of the odor sensor 220 (S204).

To this end, the controller 250 performs control to turn on the first heater 230-1, such that the odor sensor 220 may be heated, as illustrated in FIG. 8.

In this case, when the odor sensor 220 is heated, the previous odor measurement target gas particles may be easily desorbed from the surface of the odor sensor 220 (S205). This is because the adsorption binding force of the gas particles to the surface of the odor sensor decreases as the momentum of the gas particles increases due to thermal vibration, as described above.

Moreover, the previous odor measurement target gas particles may be more easily desorbed from the surface of the odor sensor 220 by a flow force of the heated cleaning air flowing in the odor sensor chamber 210.

Therefore, the previous odor measurement target gas particles, which remain in the odor sensor chamber 210, and the previous odor measurement target gas particles, which are desorbed from the surface of the odor sensor 220, may be discharged to the outside through the second gas discharge port 212 of the odor sensor chamber 210 by the flow force of the cleaning air flowing in the odor sensor chamber 210 (S206).

Next, new odor measurement target gas is cooled in advance and supplied into the odor sensor chamber 210 (S207).

To this end, the controller 250 performs control to open the second valve 232 and close the first valve 231 and the third valve 233 to supply the new odor measurement target gas into the odor sensor chamber 210, such that the new odor measurement target gas may pass through the first gas inlet port 202 of the pre-chamber 200 and flow along the gas flow tube 204.

Particularly, the controller 250 performs fuzzy control or on/off control on the pump for supplying the new odor measurement target gas on the basis of information on a volume of the gas flow tube 204 of the pre-chamber 200, information on a volume of the odor sensor chamber 210, and information on a flow rate of the new odor measurement target gas determined in accordance with a capacity of the pump configured to supply the new odor measurement target gas, such that the new odor measurement target gas may be supplied into the odor sensor chamber 210 along the gas flow tube 204 at a flow rate corresponding to the volume of the gas flow tube 204 of the pre-chamber 200 and the volume of the odor sensor chamber 210.

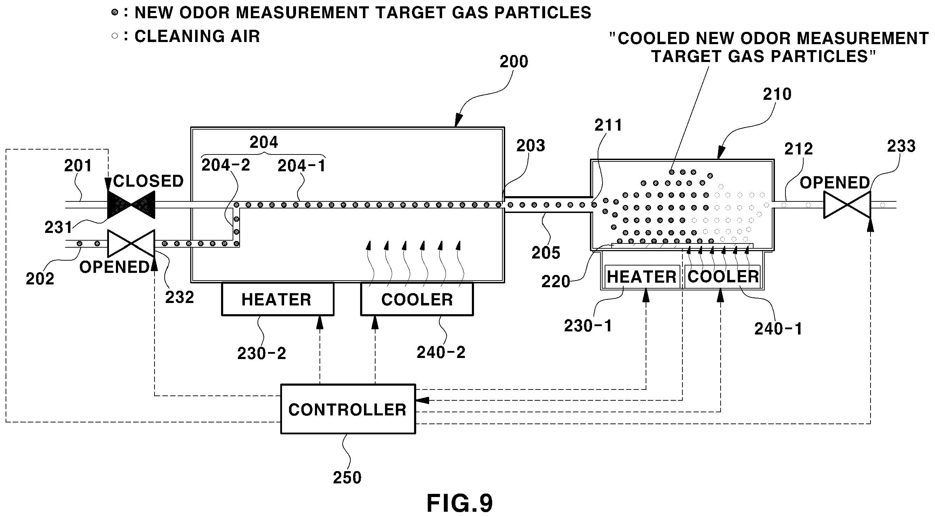

In addition, the controller 250 performs control to turn on the second cooler 240-2 mounted in the pre-chamber 200 when performing control to open the second valve 232, such that the new odor measurement target gas flowing along the gas flow tube 204 may be cooled in advance, as illustrated in FIG. 9, and the cooled new odor measurement target gas may pass sequentially through the first gas discharge port 203, the connection tube 205, and the second gas inlet port 211 and be supplied into the odor sensor chamber 210.

Therefore, when the cooled new odor measurement target gas is supplied into the odor sensor chamber 210, the new odor measurement target gas particles may be easily adsorbed to the surface of the odor sensor 220. This is because the adsorption binding force of the gas particles to the surface of the odor sensor increases as the momentum of the gas particles decreases due to thermal vibration.

In addition, the controller 250 performs control to turn on the first cooler 240-1 when performing control to open the second valve 232, such that the odor sensor 220 may be cooled, as illustrated in FIG. 9 (S208).

In this case, when the odor sensor 220 is cooled, the new odor measurement target gas particles may be more easily adsorbed to the surface of the odor sensor 220. This is because the adsorption binding force of the gas particles to the surface of the odor sensor increases as the momentum of the gas particles decreases due to thermal vibration, as described above.

Therefore, the process of cooling in advance the new odor measurement target gas and cooling the odor sensor 220 allows the new odor measurement target gas particles to be easily adsorbed to the surface of the odor sensor 220, such that the measurement accuracy of the odor sensor may be improved.

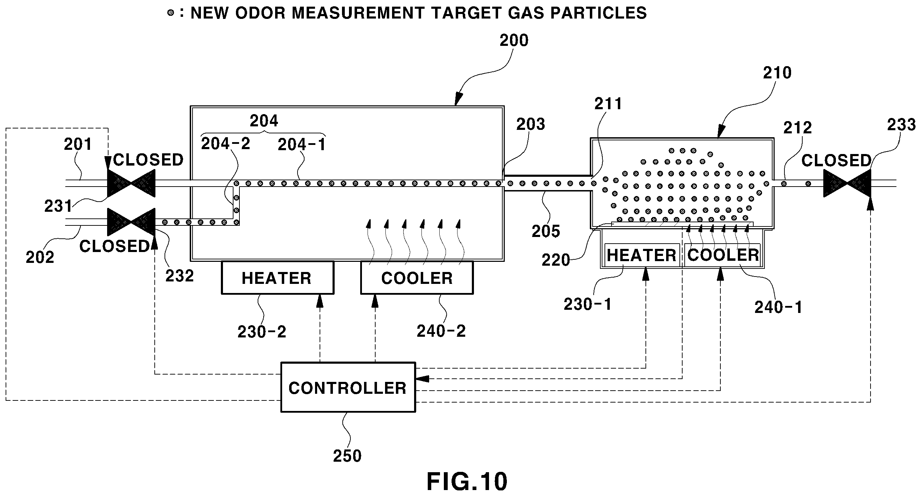

Particularly, after the new odor measurement target gas particles are stably adsorbed to the surface of the odor sensor 220 as the controller 250 performs control to turn on the first cooler 240-1 and the second cooler 240-2 for a predetermined time, the controller 250 performs control to close the first valve 231, the second valve 232, and the third valve 233, as illustrated in FIG. 10, such that only the new odor measurement target gas remains in the odor sensor chamber 210, and the odor measurement may be accurately performed by the odor sensor 220.

According to another embodiment of the present disclosure described above, the cleaning air, which is heated in advance, is supplied into the odor sensor chamber 210, such that the cleaning air, which is heated in advance, eliminates the previous odor measurement target gas particles remaining in the odor sensor chamber 210 and remaining on the surface of the odor sensor 220. The process of heating the odor sensor 220 allows the previous odor measurement target gas particles to be easily desorbed from the surface of the odor sensor 220. Next, the process of cooling the odor sensor 220 while cooling in advance the new odor measurement target gas and supplying the new odor measurement target gas into the odor sensor chamber 210 allows the new odor measurement target gas particles to be easily adsorbed to the surface of the odor sensor 220. Therefore, it is possible to improve the measurement accuracy of the odor sensor 220.

Meanwhile, the odor measurement device of embodiments of the present disclosure may be mounted in future mobility vehicles such as purpose-built vehicles (PBVs) and urban air mobility (UAM) vehicles as well as the vehicles and measure various types of odors produced in the vehicles and mobility vehicles. Therefore, the odor measurement device may clearly analyze the causes of the actual offensive odor on the basis of the measured odor data and contribute to the improvement on the odor-related functional quality of various types of vehicles and future mobility vehicles.

In addition, the odor measurement device of embodiments of the present disclosure may be mounted on robots that may easily approach various types of industrial sites (e.g., industrial sites in which gases leak or offensive odors leak). Therefore, it is possible to greatly improve the utilization of the robot by accurately measuring components and concentrations of the odors produced in various types of industrial sites.

While embodiments of the present disclosure have been described in detail with reference to several exemplary embodiments, the protection scope of the present disclosure is not limited to the above-mentioned embodiments. It should be construed that many variations and modifications made by those skilled in the art using the basic concept of embodiments of the present disclosure, which are defined in the following claims, will also belong to the right scope of the present disclosure.

Embodiments of the invention have been described in detail with reference to preferred embodiments thereof. However, it will be appreciated by those skilled in the art that changes may be made in these embodiments without departing from the embodiments of the invention, the scope of which is defined in the appended claims.

## Claims

1. A device for measuring an odor, the device comprising:
an odor sensor chamber (100) comprising a cleaning air supply port (101) and a gas inlet port (102) provided at a first side portion and a gas discharge port (103) provided at a second side portion;
an odor sensor (110) mounted in the odor sensor chamber (100);
a first valve (121) mounted in the cleaning air supply port (101);
a second valve (122) mounted in the gas inlet port (102);
a third valve (123) mounted in the gas discharge port (103);
a heater (130) mounted in the odor sensor chamber (100);
a cooler (140) mounted in the odor sensor chamber (100); and
a controller (150) configured to perform control to open or close the first valve (121), the second valve (122), and the third valve (123) and to turn on or off the heater (130) and the cooler (140).

2. The device of claim 1, wherein the controller (150) is configured to perform control to open the first valve (121) and the third valve (123) and close the second valve (122) to supply cleaning air into the odor sensor chamber (100) after odor measurement by the odor sensor (110) is ended.

3. The device of claim 1 or 2, wherein the controller (150) is configured to perform control to turn on the heater (130) to heat the odor sensor (110) such that previous odor measurement target gas particles are desorbed from a surface of the odor sensor (110) after the odor measurement by the odor sensor (110) is ended.

4. The device of anyone of claims 1-3, wherein the controller (150) is configured to perform control to open the second valve (122) and close the first valve (121) and the third valve (123) to supply a new odor measurement target gas into the odor sensor chamber (100).

5. The device of anyone of claims 1-4, wherein the controller (150) is configured to perform control to turn on the cooler to cool the odor sensor (110) such that particles of the new odor measurement target gas are adsorbed to a surface of the odor sensor (110).

6. A method of measuring an odor using the device of anyone of claims 1-5, the method comprising:
ending odor measurement by the odor sensor (110) mounted in the odor sensor chamber (100);
supplying cleaning air into the odor sensor chamber (100) to eliminate previous odor measurement target gas particles;
heating the odor sensor (110) by turning on a first heater (130) mounted in the odor sensor chamber (100) to desorb the previous odor measurement target gas particles from a surface of the odor sensor (110); and
discharging the previous odor measurement target gas particles to an outside of the odor sensor chamber (100) by using the cleaning air.

7. The method of claim 6, further comprising cooling the odor sensor (110) by turning on the cooler mounted in the odor sensor chamber (100) such that new odor measurement target gas particles are adsorbed to the surface of the odor sensor (110) when new odor measurement target gas is supplied into the odor sensor chamber (100).

## Patentansprüche

1. Vorrichtung zum Messen eines Geruchs, wobei die Vorrichtung aufweist:
eine Geruchssensorkammer (100) aufweisend eine Reinigungsluftzuführöffnung (101) und eine Gaseinlassöffnung (102), die an einem ersten Seitenabschnitt vorgesehen sind, und eine Gasauslassöffnung (103), die an einem zweiten Seitenabschnitt vorgesehen ist,
einen Geruchssensor (110), der in der Geruchssensorkammer (100) montiert ist,
ein erstes Ventil (121), das in der Reinigungsluftzuführöffnung (101) montiert ist,
ein zweites Ventil (122), das in der Gaseinlassöffnung (102) montiert ist,
ein drittes Ventil (123), das in der Gasauslassöffnung (103) montiert ist,
eine Heizvorrichtung (130), die in der Geruchssensorkammer (100) montiert ist,
einen Kühler (140), der in der Geruchssensorkammer (100) montiert ist, und
eine Steuereinrichtung (150), die konfiguriert ist, um eine Steuerung durchzuführen, um das erste Ventil (121), das zweite Ventil (122) und das dritte Ventil (123) zu öffnen oder zu schließen, und um die Heizvorrichtung (130) und den Kühler (140) einzuschalten oder auszuschalten.

2. Vorrichtung gemäß Anspruch 1, wobei die Steuereinrichtung (150) konfiguriert ist, um eine Steuerung durchzuführen, um das erste Ventil (121) und das dritte Ventil (123) zu öffnen und das zweite Ventil (122) zu schließen, um Reinigungsluft in die Geruchssensorkammer (100) zuzuführen, nachdem eine Geruchsmessung durch den Geruchssensor (110) beendet ist.

3. Vorrichtung gemäß Anspruch 1 oder 2, wobei die Steuereinrichtung (150) konfiguriert ist, um eine Steuerung durchzuführen, um die Heizvorrichtung (130) einzuschalten, um den Geruchssensor (110) zu erwärmen, so dass Vorheriges-Geruchsmessungs-Zielgas-Partikel nach Beendigung der Geruchsmessung durch den Geruchssensor (110) von einer Fläche des Geruchssensors (110) desorbiert werden.

4. Vorrichtung gemäß irgendeinem der Ansprüche 1 bis 3, wobei die Steuereinrichtung (150) konfiguriert ist, um eine Steuerung durchzuführen, um das zweite Ventil (122) zu öffnen und das erste Ventil (121) und das dritte Ventil (123) zu schließen, um ein neues Geruchsmessungs-Zielgas in die Geruchssensorkammer (100) zuzuführen.

5. Vorrichtung gemäß irgendeinem der Ansprüche 1 bis 4, wobei die Steuereinrichtung (150) konfiguriert ist, um eine Steuerung durchzuführen, um den Kühler einzuschalten, um den Geruchssensor (110) zu kühlen, so dass Partikel des neuen Geruchsmessungs-Zielgases an einer Fläche des Geruchssensors (110) adsorbiert werden.

6. Verfahren zum Messen eines Geruchs unter Verwendung der Vorrichtung gemäß irgendeinem der Ansprüche 1 bis 5, wobei das Verfahren aufweist:
Beenden einer Geruchsmessung durch den in der Geruchssensorkammer (100) montierten Geruchssensor (110),
Zuführen von Reinigungsluft in die Geruchssensorkammer (100), um Vorheriges-Geruchsmessungs-Zielgas-Partikel zu entfernen,
Erwärmen des Geruchssensors (110) durch Einschalten einer ersten Heizvorrichtung (130), die in der Geruchssensorkammer (100) montiert ist, um die Vorheriges-Geruchsmessungs-Zielgas-Partikel von einer Fläche des Geruchssensors (110) zu desorbieren, und
Ableiten der Vorheriges-Geruchsmessungs-Zielgas-Partikel zu einem Äußeren der Geruchssensorkammer (100) unter Verwendung der Reinigungsluft.

7. Verfahren gemäß Anspruch 6, ferner aufweisend das Kühlen des Geruchssensors (110) durch Einschalten des in der Geruchssensorkammer (100) montierten Kühlers, so dass Neues-Geruchsmessungs-Zielgas-Partikel an der Fläche des Geruchssensors (110) adsorbiert werden, wenn neues Geruchsmessungs-Zielgas in die Geruchssensorkammer (100) zugeführt wird.

## Revendications

1. Dispositif pour mesurer une odeur, le dispositif comprenant :
une chambre de capteur d'odeur (100) comprenant un orifice d'alimentation en air de nettoyage (101) et un orifice d'entrée de gaz (102) prévus sur une première partie latérale, et un orifice d'évacuation de gaz (103) prévu sur une deuxième partie latérale ;
un capteur d'odeur (110) monté dans la chambre de capteur d'odeur (100) ;
une première vanne (121) montée dans l'orifice d'alimentation en air de nettoyage (101) ;
une deuxième vanne (122) montée dans l'orifice d'entrée de gaz (102) ;
une troisième vanne (123) montée dans l'orifice d'évacuation de gaz (103) ;
un dispositif de chauffage (130) monté dans la chambre de capteur d'odeur (100) ;
un refroidisseur (140) monté dans la chambre de capteur d'odeur (100) ; et
un contrôleur (150) configuré pour effectuer une commande pour ouvrir ou fermer la première vanne (121), la deuxième vanne (122) et la troisième vanne (123), et pour mettre en marche ou arrêter le dispositif de chauffage (130) et le refroidisseur (140).

2. Dispositif selon la revendication 1, dans lequel le contrôleur (150) est configuré pour effectuer une commande pour ouvrir la première vanne (121) et la troisième vanne (123) et fermer la deuxième vanne (122) afin de fournir de l'air de nettoyage dans la chambre de capteur d'odeur (100) une fois qu'une mesure d'odeur par le capteur d'odeur (110) est terminée.

3. Dispositif selon la revendication 1 ou 2, dans lequel le contrôleur (150) est configuré pour effectuer une commande pour mettre en marche le dispositif de chauffage (130) afin de chauffer le capteur d'odeur (110) de telle sorte que des particules de gaz cible de mesure d'odeur précédent soient désorbées d'une surface du capteur d'odeur (110) une fois la mesure d'odeur effectuée par le capteur d'odeur (110) terminée.

4. Dispositif selon l'une quelconque des revendications 1 à 3, dans lequel le contrôleur (150) est configuré pour effectuer une commande pour ouvrir la deuxième vanne (122) et fermer la première vanne (121) et la troisième vanne (123) afin de fournir un nouveau gaz cible de mesure d'odeur dans la chambre de capteur d'odeur (100).

5. Dispositif selon l'une quelconque des revendications 1 à 4, dans lequel le contrôleur (150) est configuré pour effectuer une commande pour mettre en marche le refroidisseur afin de refroidir le capteur d'odeur (110) de telle sorte que des particules du nouveau gaz cible de mesure d'odeur soient adsorbées sur une surface du capteur d'odeur (110).

6. Procédé de mesure d'une odeur en utilisant le dispositif selon l'une quelconque des revendications 1 à 5, le procédé comprenant :
la fin de mesure d'odeur par le capteur d'odeur (110) monté dans la chambre de capteur d'odeur (100) ;
la fourniture d'air de nettoyage dans la chambre de capteur d'odeur (100) afin d'éliminer des particules de gaz cible de mesure d'odeur précédent ;
le chauffage du capteur d'odeur (110) en mettant en marche un premier dispositif de chauffage (130) monté dans la chambre de capteur d'odeur (100) afin de désorber les particules de gaz cible de mesure d'odeur précédent d'une surface du capteur d'odeur (110) ; et
l'évacuation des particules de gaz cible de mesure d'odeur précédent vers un extérieur de la chambre de capteur d'odeur (100) en utilisant l'air de nettoyage.

7. Procédé selon la revendication 6, comprenant en outre le refroidissement du capteur d'odeur (110) en mettant en marche le refroidisseur monté dans la chambre de capteur d'odeur (100) de telle sorte que des particules de nouveau gaz cible de mesure d'odeur soient adsorbées sur la surface du capteur d'odeur (110) lorsque du nouveau gaz cible de mesure d'odeur est introduit dans la chambre de capteur d'odeur (100).
